# EUROPEAN PATENT SPECIFICATION

(11) **EP 4 551 302 B1**
(45) Date of publication and mention of the grant of the patent: **20.08.2025**
(21) Application number: 24765860.2
(22) Date of filing: 15.08.2024
(51) Int. Cl.: A61Q 5/12, A61K 8/34, A61K 8/41

(54) **HAIR CARE COMPOSITION**
HAARPFLEGEMITTEL
COMPOSITION DE SOINS CAPILLAIRES

(30) Priority: 15.08.2023 US 202363519571 P
(43) Date of publication of application: 14.05.2025
(73) Proprietor: The Procter & Gamble Company, Cincinnati, OH 45202 (US)
(72) Inventor: SOH, Mui Siang, Singapore 138547 (SG); KANG, Husen,Kartasasmita, Singapore 138547 (SG)
(74) Representative: P&G Patent Germany
(86) International application number: PCT/US2024/042415
(87) International publication number: WO 2025/038809

(56) References cited:
- US-A1- 2013 259 817
- US-A1- 2021 161 784

## Description

### FIELD OF THE INVENTION

The present invention relates to a hair care composition comprising a fatty alcohol, high levels of cationic surfactant, and an aqueous carrier. The compositions of the present invention provide improved conditioning on damaged hair without greasy or weighed down hair, while providing improved deposition of silicone compounds.

### BACKGROUND OF THE INVENTION

A variety of approaches have been developed to condition the hair. A common method of providing conditioning benefit is through the use of conditioning agents such as cationic surfactants and polymers, high melting point fatty compounds, low melting point oils, silicone compounds, and mixtures thereof. Most of these conditioning agents are known to provide various conditioning benefits.

Typically, the level of fatty compounds is greater than the level of cationic surfactants. The high level of fatty compounds will lead to excess deposition on the hair. Through multi cycles use, the fatty compounds will build-up on hair and lead to weigh down hair and make hair look greasy and heavy. In order not to have hair that looks heavy and greasy, composition that containers lower amount of conditioning actives can be used. However, the composition will not provide enough conditioning for high conditioning seeker while the use of higher amounts of conditioning agents will lead to weigh down hair. In both scenarios, excess deposition of fatty compounds remained. This combination of levels of fatty compounds greater than levels of cationic surfactants means the composition will deposit more on the roots of the hair and the hair becomes less clean or fails to have good volume.

US 2013/259817 A1 discloses a hair conditioning composition comprising (a) from about 0.1% to about 8% of a mono-alkyl amine cationic surfactant which is a primary, secondary, and tertiary amines having one long alkyl or alkenyl group of from about 12 to about 30 carbon atoms; (b) from about 0.05% to about 6% of a di-alkyl quaternized ammonium salt cationic surfactant having two long alkyl groups of from about 12 to about 30 carbon atoms; (c) from about 1% to about 15% of a high melting point fatty compound; (d) from about 0.05% to about 6% of a deposition polymer which is a copolymer comprising: a vinyl monomer (A) with a carboxyl group in the structure; and a vinyl monomer, (e) from about 0.05% to about 15% of a silicone compound; and (f) an aqueous carrier. A gel matrix is formed by the cationic surfactants, the high melting point fatty compound, and an aqueous carrier. The gel matrix is suitable for providing various conditioning benefits, such as slippery feel during the application to wet hair and softness and moisturized feel on dry hair. When the gel matrix is formed, the cationic surfactant and the high melting point fatty compound are contained at a level such that the weight ratio of the cationic surfactant to the high melting point fatty compound is in the range of, preferably from about 1:1 to about 1:10, more preferably from about 1:1.5 to about 1:7, still more preferably from about 1:2 to about 1:6, in view of providing improved wet conditioning benefits. Preferably, when the gel matrix is formed, the composition of the present invention is substantially free of anionic surfactants.

Thus, there remains a need for a hair care composition that does not lead to excess deposition on the roots of hair and yet provides conditioning benefits to those who perceive that they have greasy roots and dry tips.

### SUMMARY OF THE INVENTION

The scope of protection is defined by the claims.

Disclosed herein is a hair care composition comprising a fatty alcohol, a cationic surfactant and an aqueous base. The weight ratio of the fatty alcohol to the cationic surfactant is from 1:2 to 1:20, and the composition is free of anionic surfactant.

### DETAILED DESCRIPTION OF THE INVENTION

While the specification concludes with claims particularly pointing out and distinctly claiming the invention, it is believed that the present invention will be better understood from the following description.

Herein, "comprising" means that other steps and other ingredients which do not affect the end result can be added. This term encompasses the terms "consisting of" and "consisting essentially of".

All percentages, parts and ratios are based upon the total weight of the compositions of the present invention, unless otherwise specified. All such weights as they pertain to listed ingredients are based on the active level and, therefore, do not include carriers or by-products that may be included in commercially available materials.

Herein, "mixtures" is meant to include a simple combination of materials and any compounds that may result from their combination.

The term "molecular weight" or "M.Wt." as used herein refers to the weight average molecular weight unless otherwise stated. The weight average molecular weight may be measured by gel permeation chromatography.

"QS" means sufficient quantity for 100%.

### HAIR CARE COMPOSITIONS

The present hair care compositions comprise a fatty alcohol, a cationic surfactant, and an aqueous base, wherein the ratio of the fatty alcohol to the cationic surfactant may be 1:2 to 1:20, and in other embodiments from 1:2 to 1:10, or from 1:2 to 1:9, or from 1:2 to 1:8, or from 1:2 to 1:6; and in other embodiments from 1:2.5 to 1:10, or from 1:2.5 to 1:8, or from 1:2.5 to 1: 7, or from 1:3 to 1:10.

Conventional conditioners are typically formulated with high amounts of fatty alcohol (FA) and low amounts of cationic surfactant (CS) for reduction in wet combing forces on hair and reduced surface friction etc. In addition to fatty alcohol and cationic surfactant, silicone is also often added as a conditioning agent for smooth and manageable hair. Silicone and fatty alcohol are hydrophobic in nature and tend to deposit more at the hydrophobic part of the hair and often lead to over conditioning at the root. This over-deposition may be the cause for increased inter-fiber adhesion due to greater sebum affinity (sebum is also hydrophobic). Thus, this invention aims to modify the hair surfaces through the use of high amounts of cationic surfactant to increase silicone deposition on damaged hair and to decrease fatty alcohol deposition on healthy hair. If silicone deposition can be increased on damaged hair, damaged hair conditioning can be increased. This increase may lead to indirect silicone reduction at the roots and hence result in less clumpy roots.

### HIGH MELTING POINT FATTY ALCOHOL

The compositions of the present invention comprise a high melting point fatty alcohol. The high melting point fatty alcohol is included in the composition at a level of preferably from 0.05% to 12.5%, or from 0.1% to 20%, or from 0.25% to 10%, or from 0.25% to 8%, or from 0.5% to 6%, or from 0.5% to 3%, or from 0.5% to 2%, or from 0.1% to 17%, or from 0.1% to 15%, or from 0.1% to 12% by weight of the composition, in view of providing improved conditioning benefits such as slippery feel during the application to wet hair, softness and moisturized feel on dry hair.

The high melting point fatty alcohol useful herein have a melting point of 25°C or higher. Further, it is understood by the artisan that, depending on the number and position of double bonds, and length and position of the branches, certain compounds having certain required carbon atoms may have a melting point of less than 25°C. Such compounds of low melting point are not intended to be included in this section. Nonlimiting examples of the high melting point compounds are found in International Cosmetic Ingredient Dictionary, Fifth Edition, 1993, and CTFA Cosmetic Ingredient Handbook, Second Edition, 1992. The fatty alcohols useful herein are those having from 12 to 30 carbon atoms, preferably from 16 to 22 carbon atoms. These fatty alcohols are saturated and can be straight or branched chain alcohols. Preferred fatty alcohols include, for example, cetyl alcohol, stearyl alcohol, behenyl alcohol, and mixtures thereof.

High melting point fatty alcohols of a single compound of high purity are preferred. Single compounds of pure fatty alcohols selected from the group of pure cetyl alcohol, stearyl alcohol, and behenyl alcohol are highly preferred. By "pure" herein, what is meant is that the compound has a purity of at least 90%, preferably at least 95%. These single fatty alcohols compounds of high purity provide good rinsability from the hair when the consumer rinses off the composition.

In the present invention, a more preferred fatty alcohol is a mixture of cetyl alcohol and stearyl alcohol.

Generally, in the mixture, the weight ratio of cetyl alcohol to stearyl alcohol is preferably from 1:9 to 9:1, more preferably from 1:4 to 4:1, still more preferably from 1:2.3 to 1.5:1.

When using higher levels of total cationic surfactant and high melting point fatty alcohols the mixture has the weight ratio of cetyl alcohol to stearyl alcohol of preferably from 1:1 to 4:1, more preferably from 1:1 to 2:1, still more preferably from 1.2:1 to 2:1, in view to avoid getting too thick for spreadability. It may also provide more conditioning on damaged part of the hair.

### CATIONIC SURFACTANT

The compositions of the present invention comprise a cationic surfactant. The cationic surfactant can be included in the composition at a level of from 0.1% to 25%, more preferably from 0.5% to 20%, still more preferably from 0.5% to 16%, still more preferably from 1% to 15%, still more preferably from 1 % to 14%. by weight of the composition, in view of providing the benefits of the present invention.

For purposes of calculating the weight percent of cationic surfactant, either as a part of the total composition, or when calculating the ratio of fatty alcohol to cationic surfactant, the amount of active cationic surfactant should be used. In many cases, commercially-bought cationic surfactants may be sold with by-products, such as isopropyl alcohol, thus the active amount of cationic surfactant may be, for example, 80% of the amount of cationic surfactant as bought and added to the inventive composition.

Preferably, in the present invention, the surfactant is water-insoluble. In the present invention, "water-insoluble surfactants" means that the surfactants have a solubility in water at 25°C of preferably below 0.5g/100g (excluding 0.5g/100g) water, more preferably 0.3g/100g water or less.

Cationic surfactant useful herein can be one cationic surfactant or a mixture of two or more cationic surfactants. Preferably, the cationic surfactant is selected from: a mono-long alkyl quaternized ammonium salt; a combination of a mono-long alkyl quaternized ammonium salt and a di-long alkyl quaternized ammonium salt; a mono-long alkyl amine; a combination of a mono-long alkyl amine and a di-long alkyl quaternized ammonium salt; and a combination of a mono-long alkyl amine and a mono-long alkyl quaternized ammonium salt.

### Mono-long alkyl amine

Mono-long alkyl amine useful herein are those having one long alkyl chain of preferably from 12 to 30 carbon atoms, more preferably from 16 to 24 carbon atoms, still more preferably from 18 to 22 alkyl group. Mono-long alkyl amines useful herein also include mono-long alkyl amidoamines. Primary, secondary, and tertiary fatty amines are useful.

Particularly useful are tertiary amido amines having an alkyl group of from about 12 to about 22 carbons. Exemplary tertiary amido amines include: stearamidopropyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, arachidamidopropyldimethylamine, arachidamidopropyldiethylamine, arachidamidoethyldiethylamine, arachidamidoethyldimethylamine, diethylaminoethylstearamide. Useful amines in the present invention are disclosed in U.S. Patent 4,275,055, Nachtigal, et al.

These amines are used in combination with acids such as L-glutamic acid, lactic acid, hydrochloric acid, malic acid, succinic acid, acetic acid, fumaric acid, tartaric acid, citric acid, L-glutamic hydrochloride, maleic acid, salicylic acid, aspartic acid and mixtures thereof; more preferably L-glutamic acid, lactic acid, citric acid, at a molar ratio of the amine to the acid of from 1:0.3 to 1:5, more preferably from 1:0.4 to 1:2 and more preferably from 1:0.4 to 1:1..

### Mono-long alkyl quaternized ammonium salt

The mono-long alkyl quaternized ammonium salts useful herein are those having one long alkyl chain which has from 12 to 30 carbon atoms, preferably from 16 to 24 carbon atoms, more preferably C18-22 alkyl group. The remaining groups attached to nitrogen are independently selected from an alkyl group of from 1 to 4 carbon atoms or an alkoxy, polyoxyalkylene, alkylamido, hydroxyalkyl, aryl or alkylaryl group having up to 4 carbon atoms

Mono-long alkyl quaternized ammonium salts useful herein are those having the formula (I): 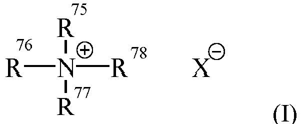 wherein one of R⁷⁵, R⁷⁶, R⁷⁷ and R⁷⁸ is selected from an alkyl group of from 12 to 30 carbon atoms or an aromatic, alkoxy, polyoxyalkylene, alkylamido, hydroxyalkyl, aryl or alkylaryl group having up to 30 carbon atoms; the remainder of R⁷⁵, R⁷⁶, R⁷⁷ and R⁷⁸ are independently selected from an alkyl group of from 1 to 4 carbon atoms or an alkoxy, polyoxyalkylene, alkylamido, hydroxyalkyl, aryl or alkylaryl group having up to 4 carbon atoms; and X⁻ is a salt-forming anion such as those selected from halogen, (e.g. chloride, bromide), acetate, citrate, lactate, glycolate, phosphate, nitrate, sulfonate, sulfate, alkylsulfate, and alkyl sulfonate radicals. The alkyl groups can contain, in addition to carbon and hydrogen atoms, ether and/or ester linkages, and other groups such as amino groups. The longer chain alkyl groups, e.g., those of 12 carbons, or higher, can be saturated or unsaturated. Preferably, one of R⁷⁵, R⁷⁶, R⁷⁷ and R⁷⁸ is selected from an alkyl group of from 12 to 30 carbon atoms, more preferably from 16 to 24 carbon atoms, still more preferably from 18 to 22 carbon atoms, even more preferably 22 carbon atoms; the remainder of R⁷¹, R⁷⁶, R⁷⁷ and R⁷⁸ are independently selected from CH₃, C₂H₅, C₂H₄OH, and mixtures thereof; and X is selected from the group consisting of Cl, Br, CH₃OSO₃, C₂H₅OSO₃, and mixtures thereof.

Nonlimiting examples of such mono-long alkyl quaternized ammonium salt cationic surfactants include: behenyl trimethyl ammonium salt; stearyl trimethyl ammonium salt; cetyl trimethyl ammonium salt; and hydrogenated tallow alkyl trimethyl ammonium salt.

### Di-long alkyl quaternized ammonium salts

When used, di-long alkyl quaternized ammonium salts are preferably combined with a mono-long alkyl quaternized ammonium salt and/or mono-long alkyl amine salt, at the weight ratio of from 1:1 to 1:5, more preferably from 1:1.2 to 1:5, still more preferably from 1:1.5 to 1:4, in view of stability in rheology and conditioning benefits.

Di-long alkyl quaternized ammonium salts useful herein are those having two long alkyl chains of from 12 to 30 carbon atoms, more preferably from 16 to 24 carbon atoms, still more preferably from 18 to 22 carbon atoms. Such di-long alkyl quaternized ammonium salts useful herein are those having the formula (I): 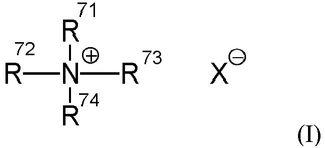 wherein two of R⁷¹, R⁷², R⁷³ and R⁷⁴ are selected from an aliphatic group of from 12 to 30 carbon atoms, preferably from 16 to 24 carbon atoms, more preferably from 18 to 22 carbon atoms or an aromatic, alkoxy, polyoxyalkylene, alkylamido, hydroxyalkyl, aryl or alkylaryl group having up to 30 carbon atoms; the remainder of R⁷¹, R⁷², R⁷³ and R⁷⁴ are independently selected from an aliphatic group of from 1 to 8 carbon atoms, preferably from 1 to 3 carbon atoms or an aromatic, alkoxy, polyoxyalkylene, alkylamido, hydroxyalkyl, aryl or alkylaryl group having up to 8 carbon atoms; and X⁻ is a salt-forming anion selected from the group consisting of halides such as chloride and bromide, C1-C4 alkyl sulfate such as methosulfate and ethosulfate, and mixtures thereof. The aliphatic groups can contain, in addition to carbon and hydrogen atoms, ether linkages, and other groups such as amino groups. The longer chain aliphatic groups, e.g., those of 16 carbons, or higher, can be saturated or unsaturated. Preferably, two of R⁷¹, R⁷², R⁷³ and R⁷⁴ are selected from an alkyl group of from 12 to 30 carbon atoms, preferably from 16 to 24 carbon atoms, more preferably from 18 to 22 carbon atoms; and the remainder of R⁷¹, R⁷², R⁷³ and R⁷⁴ are independently selected from CH₃, C₂H₅, C₂H₄OH, CH₂C₆H₅, and mixtures thereof.

Such preferred di-long alkyl cationic surfactants include, for example, dialkyl (14-18) dimethyl ammonium chloride, ditallow alkyl dimethyl ammonium chloride, dihydrogenated tallow alkyl dimethyl ammonium chloride, distearyl dimethyl ammonium chloride, and dicetyl dimethyl ammonium chloride.

### AQUEOUS CARRIER

The composition of the present invention comprises an aqueous carrier. The level and species of the carrier are selected according to the compatibility with other components, and other desired characteristic of the product.

The carrier useful in the present invention includes water and water solutions of lower alkyl alcohols. The lower alkyl alcohols useful herein are monohydric alcohols having 1 to 6 carbons, more preferably ethanol and isopropanol.

Preferably, the aqueous carrier is substantially water. Deionized water is preferably used. Water from natural sources including mineral cations can also be used, depending on the desired characteristic of the product. Generally, the compositions of the present invention comprise from from 50% to 95%, and more preferably from 70% to 90%, and more preferably from 80% to 90% aqueous carrier, preferably water.

### GEL MATRIX

Preferably, in the present invention, a gel matrix is formed by the cationic surfactant, the high melting point fatty compound, and an aqueous carrier. The gel matrix is suitable for providing various conditioning benefits, such as slippery feel during the application to wet hair and softness and moisturized feel on dry hair. The gel matrix here refers to a lamellar gel network, lamellar or vesicular solid crystalline, unilamellar vesicles, multilamellar vesicles and mixtures thereof.

Preferably, when the gel matrix is formed, the cationic surfactant and the high melting point fatty compound are contained at a level such that the weight ratio of the cationic surfactant to the high melting point fatty compound is in the range of, preferably from 2:1 20:1, more preferably from 2:1 to 10:1, still more preferably from 2.5:1 to 9:1, still more preferably from 2.5:1 to 7:1 in view of providing improved wet conditioning benefits.

Preferably, when the gel matrix is formed, the composition of the present invention is free of anionic surfactants, in view of stability of the gel The total level of such anionic surfactants is 0% by weight of the composition.

### SILICONE COMPOUND

The compositions of the present invention may further contain a silicone compound. It is believed that the silicone compound can provide smoothness and softness on dry hair. The silicone compounds herein can be used at levels by weight of the composition of preferably from 0.1% to 20%, more preferably from 0.5% to 10%, still more preferably from 1% to 8%.

Preferably, the silicone compounds have an average particle size of from about 1 micron to about 50 microns, in the composition.

The silicone compounds useful herein, as a single compound, as a blend or mixture of at least two silicone compounds, or as a blend or mixture of at least one silicone compound and at least one solvent, have a viscosity of preferably from about 1,000 to about 2,000,000mPa·s at 25°C.

The viscosity can be measured by means of a glass capillary viscometer as set forth in Dow Corning Corporate Test Method CTM0004, July 20, 1970. Suitable silicone fluids include polyalkyl siloxanes, polyaryl siloxanes, polyalkylaryl siloxanes, polyether siloxane copolymers, amino substituted silicones, quaternized silicones, and mixtures thereof. Other nonvolatile silicone compounds having conditioning properties can also be used.

Preferred polyalkyl siloxanes include, for example, polydimethylsiloxane, polydiethylsiloxane, and polymethylphenylsiloxane. Polydimethylsiloxane, which is also known as dimethicone, is especially preferred.

The above polyalkylsiloxanes are available, for example, as a mixture with silicone compounds having a lower viscosity. Such mixtures have a viscosity of preferably from 1,000mPa·s to 100,000mPa·s, more preferably from 5,000mPa·s to 50,000mPa·s. Such mixtures preferably comprise: (i) a first silicone having a viscosity of from 100,000mPa·s to 30,000,000mPa· s at 25°C, preferably from 100,000mPa· s to 20,000,000mPa·s; and (ii) a second silicone having a viscosity of from 5mPa·s to 10,000mPa· s at 25°C, preferably from 5mPa·s to 5,000mPa·s. Such mixtures useful herein include, for example, a blend of dimethicone having a viscosity of 18,000,000mPa·s and dimethicone having a viscosity of 200mPa·s available from GE Toshiba, and a blend of dimethicone having a viscosity of 18,000,000mPa·s and cyclopentasiloxane available from GE Toshiba.

The silicone compounds useful herein also include a silicone gum. The term "silicone gum", as used herein, means a polyorganosiloxane material having a viscosity at 25°C of greater than or equal to 1,000,000 mPa.s (1,000,000 centistokes). It is recognized that the silicone gums described herein can also have some overlap with the above-disclosed silicone compounds. This overlap is not intended as a limitation on any of these materials. The "silicone gums" will typically have a mass molecular weight in excess of 200,000, generally between 200,000 and 1,000,000. Specific examples include polydimethylsiloxane, poly(dimethylsiloxane methylvinylsiloxane) copolymer, poly(dimethylsiloxane diphenylsiloxane methylvinylsiloxane) copolymer and mixtures thereof. The silicone gums are available, for example, as a mixture with silicone compounds having a lower viscosity. Such mixtures useful herein include, for example, Gum/Cyclomethicone blend available from Shin-Etsu.

Silicone compounds useful herein also include amino substituted materials. Preferred aminosilicones include, for example, those which conform to the general formula (I):

(R₁)ₐG₃₋ₐ-Si-(-OS G₂)ₙ-(-OSiG_{b}(R₁)_{2-b})ₘ-O-SiG₃₋ₐ(R₁)ₐ

wherein G is hydrogen, phenyl, hydroxy, or C₁-C₈ alkyl, preferably methyl; a is 0 or an integer having a value from 1 to 3, preferably 1; b is 0, 1 or 2, preferably 1; n is a number from 0 to 1,999; m is an integer from 0 to 1,999; the sum of n and m is a number from 1 to 2,000; a and m are not both 0; R₁ is a monovalent radical conforming to the general formula CqH_{2q}L, wherein q is an integer having a value from 2 to 8 and L is selected from the following groups: -N(R₂)CH₂-CH₂-N(R₂)₂; -N(R₂)₂; -N(R₂)₃A ; -N(R₂)CH₂-CH₂-NR₂H₂A ; wherein R₂ is hydrogen, phenyl, benzyl, or a saturated hydrocarbon radical, preferably an alkyl radical from about C₁ to about C₂₀; A is a halide ion.

Highly preferred amino silicones are those corresponding to formula (I) wherein m=0, a=1, q=3, G=methyl, n is preferably from 1500 to 1700, more preferably 1600; and L is -N(CH₃)₂ or -NH₂, more preferably -NH₂. Another highly preferred amino silicones are those corresponding to formula (I) wherein m=0, a=1, q=3, G=methyl, n is preferably from 400 to 600, more preferably about 500; and L is -N(CH₃)₂ or -NH₂, more preferably -NH₂. Such highly preferred amino silicones can be called as terminal aminosilicones, as one or both ends of the silicone chain are terminated by nitrogen containing group.

The above aminosilicones, when incorporated into the composition, can be mixed with solvent having a lower viscosity. Such solvents include, for example, polar or non-polar, volatile or non-volatile oils. Such oils include, for example, silicone oils, hydrocarbons, and esters. Among such a variety of solvents, preferred are those selected from the group consisting of non-polar, volatile hydrocarbons, volatile cyclic silicones, non-volatile linear silicones, and mixtures thereof. The non-volatile linear silicones useful herein are those having a viscosity of from 1 to 20,000 mPa.s (1 to 20,000 centistokes) preferably from 20 to 10,000 mPa.s (20 to 10,000 centistokes) at 25°C. Among the preferred solvents, highly preferred are non-polar, volatile hydrocarbons, especially non-polar, volatile isoparaffins, in view of reducing the viscosity of the aminosilicones and providing improved hair conditioning benefits such as reduced friction on dry hair. Such mixtures have a viscosity of preferably from 1,000mPa·s to 100,000mPa·s, more preferably from 5,000mPa·s to 50,000mPa·s.

Other suitable alkylamino substituted silicone compounds include those having alkylamino substitutions as pendant groups of a silicone backbone. Highly preferred are those known as "amodimethicone". Commercially available amodimethicones useful herein include, for example, BY16-872 available from Dow Corning.

The silicone compounds may further be incorporated in the present composition in the form of an emulsion, wherein the emulsion is made my mechanical mixing, or in the stage of synthesis through emulsion polymerization, with or without the aid of a surfactant selected from anionic surfactants, nonionic surfactants, cationic surfactants, and mixtures thereof.

### Silicone Polymer Containing Quaternary Groups

Silicone compounds useful herein include, for example, a Silicone Polymer Containing Quaternary Groups comprising terminal ester groups, having a viscosity up to 100,000 mPa·s and a D block length of greater than 200 D units. Without being bound by theory, this low viscosity silicone polymer provides improved conditioning benefits such as smooth feel, reduced friction, and prevention of hair damage, while eliminating the need for a silicone blend.

Structurally, the silicone polymer is a polyorganosiloxane compound comprising one or more quaternary ammonium groups, at least one silicone block comprising greater than 200 siloxane units, at least one polyalkylene oxide structural unit, and at least one terminal ester group. In one or more embodiments, the silicone block may comprise between 300 to 500 siloxane units.

The silicone polymer is present in an amount of from 0.05% to 15%, preferably from 0.1% to 10%, more preferably from 0.15% to 5%, and even more preferably from 0.2% to 4% by weight of the composition.

In a preferred embodiment, the polyorganosiloxane compounds have the general formulas (Ia) and (Ib):

M-Y-[-(N⁺R₂-T-N⁺R₂)-Y-]ₘ-[-(NR²-A-E-A'-NR²)-Y-]ₖ-M (Ia)

M-Y-[-(N⁺R₂-T-N⁺R₂)-Y-]ₘ-[-(N⁺R²₂-A-E-A'-N⁺R²₂)-Y-]ₖ-M (Ib)

wherein:
m is > 0, preferred 0.01 to 100, more preferred 0.1 to 100, even more preferred 1 to 100, specifically 1 to 50, more specifically 1 to 20, even more specifically 1 to 10,
k is 0 or an average value of from >0 to 50, or preferably from 1 to 20, or even more preferably from 1 to 10,
M represents a terminal group, comprising terminal ester groups selected from

   -OC(O)-Z

   -OS(O)₂-Z

   -OS(O₂)O-Z

   -OP(O)(O-Z)OH

   -OP(O)(O-Z)₂

   wherein Z is selected from monovalent organic residues having up to 40 carbon atoms, optionally comprising one or more hetero atoms.
A and A' each are independently from each other selected from a single bond or a divalent organic group having up to 10 carbon atoms and one or more hetero atoms, and
E is a polyalkylene oxide group of the general formula:

   -[CH₂CH_{2O}]_{q}-[CH₂CH(CH₃)O]ᵣ-[CH₂CH(C₂H₅)O]ₛ-
wherein q=0 to 200, r=0 to 200, s=0 to 200, and q+r+s = 1 to 600.
R² is selected from hydrogen or R,
R is selected from monovalent organic groups having up to 22 carbon atoms and optionally one or more heteroatoms, and wherein the free valencies at the nitrogen atoms are bound to carbon atoms, Y is a group of the formula:

   -K-S-K- and -A-E-A'-or-A'-E-A-,

   with S= 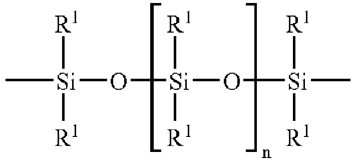 wherein R1 = C₁-C₂₂-alkyl, C₁-C₂₂-fluoralkyl or aryl; n=200 to 1000, and these can be identical or different if several S Groups are present in the polyorganosiloxane compound.
K is a bivalent or trivalent straight chain, cyclic and/or branched C₂-C₄₀ hydrocarbon residue which is optionally interrupted by-O-,-NH-, trivalent N, -NR¹-,-C(O)-, -C(S)-, and optionally substituted with-OH, wherein R¹ is defined as above,
T is selected from a divalent organic group having up to 20 carbon atoms and one or more hetero atoms.

The residues K may be identical or different from each other. In the -K-S-K- moiety, the residue K is bound to the silicon atom of the residue S via a C-Si-bond.

Due to the possible presence of amine groups (-(NR²-A-E-A'-NR²)-) in the polyorganosiloxane compounds, they may have protonated ammonium groups, resulting from the protonation of such amine groups with organic or inorganic acids. Such compounds are sometimes referred to as acid addition salts of the polyorganosiloxane compounds.

In a preferred embodiment the molar ratio of the quaternary ammonium groups b) and the terminal ester groups c) is less than 100:20, even more preferred is less than 100:30 and is most preferred less than 100:50. The ratio can be determined by ¹³C-NMR.

In a further embodiment, the polyorganosiloxane composition may comprise:
A) at least one polyorganosiloxane compound, comprising a) at least one polyorganosiloxane group, b) at least one quaternary ammonium group, c) at least one terminal ester group, and d) at least one polyalkylene oxide group (as defined before),
B) at least one polyorganosiloxane compound, comprising at least one terminal ester group, different from compound A).

In the definition of component A) it can be referred to the description of the polyorganosiloxane compounds of the invention. The polyorganosiloxane compound B) differs from the polyorganosiloxane compound A) preferably in that it does not comprise quaternary ammonium groups. Preferred polyorganosiloxane compounds B) result from the reaction of monofunctional organic acids, in particular carboxylic acids, and polyorganosiloxane containing bisepoxides.

In the polyorganosiloxane compositions the weight ratio of compound A) to compound B) is preferably less than 90:10. Or in other words, the content of component B) is at least 10 weight percent. In a further preferred embodiment of the polyorganosiloxane compositions in compound A) the molar ratio of the quaternary ammonium groups b) and the terminal ester groups c) is less than 100:10, even more preferred is less than 100: 15 and is most preferred less than 100:20.

The silicone polymer has a viscosity at 20°C and a shear rate of 0.1s⁻¹ (plate-plate system, plate diameter 40mm, gap width 0.5mm) of less than 100,000 mPa•s (100 Pa•s). In further embodiments, the viscosities of the neat silicone polymers may range from 500 to 100,000 mPa•s, or preferably from 500 to 70,000 mPa•s, or more preferably from 500 to 50,000 mPa•s, or even more preferably from 500 to 20,000 mPa•s. In further embodiments, the viscosities of the neat polymers may range from 500 to 10,000 mPa•s,or preferably 500 to 5000 mPa•s determined at 20 °C and a shear rate of 0.1 s⁻¹.

In addition to the above listed silicone polymers, the following preferred compositions are provided below. For example, in the polyalkylene oxide group E of the general formula:

-[CH₂CH₂O]_{q}-[CH₂CH(CH₃)O]ᵣ-[CH₂CH(C₂H₅)O]ₛ-

wherein the q, r, and s indices may be defined as follows:
q = 0 to 200, or preferably from 0 to 100, or more preferably from 0 to 50, or even more preferably from 0 to 20,
r = 0 to 200, or preferably from 0 to 100, or more preferably from 0 to 50, or even more preferably from 0 to 20,
s = 0 to 200, or preferably from 0 to 100, or more preferably from 0 to 50, or even more preferably from 0 to 20, and
q+r+s = 1 to 600, or preferably from 1 to 100, or more preferably from 1 to 50, or even more preferably from 1 to 40.

For polyorganosiloxane structural units with the general formula S: 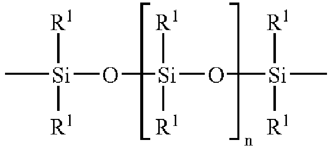 R¹=C₁-C₂₂-alkyl, C₁-C₂₂-fluoralkyl or aryl; n= from 200 to 1000, or preferably from 300 to 500, K (in the group -K-S-K-) is preferably a bivalent or trivalent straight chain, cyclical or branched C₂-C₂₀ hydrocarbon residue which is optionally interrupted by-O-,-NH-, trivalent N,-NR¹-,-C(O)-,-C(S)-, and optionally substituted with-OH.

In specific embodiments, R¹ is C₁-C₁₈ alkyl, C₁-C₁₈ fluoroalkyl and aryl. Furthermore, R¹ is preferably C₁-C₁₈ alkyl, C₁-C₆fluoroalkyl and aryl. Furthermore, R¹ is more preferably C₁-C₆ alkyl, C₁-C₆ fluoroalkyl, even more preferably C₁-C₄ fluoroalkyl, and phenyl. Most preferably, R¹ is methyl, ethyl, trifluoropropyl and phenyl.

As used herein, the term "C₁-C₂₂ alkyl" means that the aliphatic hydrocarbon groups possess from 1 to 22 carbon atoms which can be straight chain or branched. Methyl, ethyl, propyl, n-butyl, pentyl, hexyl, heptyl, nonyl, decyl, undecyl, isopropyl, neopentyl and 1,2,3-trimethyl hexyl moieties serve as examples.

Further as used herein, the term "C₁-C₂₂ fluoroalkyl" means aliphatic hydrocarbon compounds with 1 to 22 carbon atoms which can be straight chain or branched and are substituted with at least one fluorine atom. Monofluormethyl, monofluoroethyl, 1,1,1-trifluorethyl, perfluoroethyl, 1,1,1-trifluoropropyl, 1,2,2-trifluorobutyl are suitable examples.

Moreover, the term "aryl" means unsubstituted or phenyl substituted once or several times with OH, F, Cl, CF₃, C₁-C₆ alkyl, C₁-C₆ alkoxy, C₃-C₇ cycloalkyl, C₂-C₆ alkenyl or phenyl. Aryl may also mean naphthyl.

For the embodiments of the polyorganosiloxanes, the positive charges resulting from the ammonium group(s), are neutralized with inorganic anions such as chloride, bromide, hydrogen sulfate, sulfate, or organic anions, like carboxylates deriving from C₁-C₃₀ carboxylic acids, for example acetate, propionate, octanoate, especially from C₁₀-C₁₈ carboxylic acids, for example decanoate, dodecanoate, tetradecanoate, hexadecanoate, octadecanoate and oleate, alkylpolyethercarboxylate, alkylsulphonate, aryl sulphonate, alkylarylsulphonate, alkylsulphate, alkylpolyethersulphate, phosphates derived from phosphoric acid mono alkyl/aryl ester and phosphoric acid dialkyl/aryl ester. The properties of the polyorganosiloxane compounds can be, *inter alia,* modified based upon the selection of acids used.

The quaternary ammonium groups are usually generated by reacting the di-tertiary amines with an alkylating agents, selected from in particular di-epoxides (sometimes referred to also as bis-epoxides) in the presence of mono carboxylic acids and difunctional dihalogen alkyl compounds.

In a preferred embodiment the polyorganosiloxane compounds are of the general formulas (Ia) and (Ib):

M-Y-[-(N⁺R₂-T-N⁺R₂)-Y-]ₘ-[-(NR²-A-E-A'-NR²)-Y-]ₖ-M (Ia)

M-Y-[-(N⁺R₂-T-N⁺R₂)-Y-]ₘ-[-(N⁺R²₂-A-E-A'-N⁺R²₂)-Y-]ₖ-M (Ib)

wherein each group is as defined above; however, the repeating units are in a statistical arrangement (i.e., not a block-wise arrangement).

In a further preferred embodiment the polyorganosiloxane compounds may be also of the general formulas (IIa) or (IIb):

M-Y-[-N⁺R₂-Y-]ₘ-[-(NR²-A-E-A'-NR²)-Y-]ₖ-M (IIa)

M-Y-[-N⁺R₂-Y-]ₘ-[-(N⁺R²₂-A-E-A'-N⁺R²₂)-Y-]ₖ-M (IIb)

wherein each group is as defined above. Also in such formula the repeating units are usually in a statistical arrangement (i.e. not a block-wise arrangement).
wherein, as defined above, M is

-OC(O)-Z,

-OS(O)₂-Z

-OS(O₂)O-Z

-OP(O)(O-Z)OH

-OP(O)(O-Z)₂

Z is a straight chain, cyclic or branched saturated or unsaturated C₁-C₂₀, or preferably C₂ to C₁₈, or even more preferably a hydrocarbon radical, which can be interrupted by one or more - O-, or -C(O)- and substituted with -OR. In a specific embodiment, M is -OC(O)-Z resulting from normal carboxylic acids in particular with more than 10 carbon atoms like for example dodecanoic acid.

In a further embodiment, the molar ratio of the polyorganosiloxane-containing repeating group -K-S-K-and the polyalkylene repeating group -A-E-A'- or -A'-E-A- is between 100:1 and 1:100, or preferably between 20:1 and 1:20, or more preferably between 10:1 and 1:10.

In the group -(N⁺R₂-T-N⁺R₂)-, R may represent a monovalent straight chain, cyclic or branched C₁-C₂₀ hydrocarbon radical, which can be interrupted by one or more -O- , - C(O)- and can be substituted by-OH, T may represent a divalent straight-chain, cyclic, or branched C₁-C₂₀ hydrocarbon radical, which can be interrupted by -O- , -C(O)- and can be substituted by hydroxyl.

The above described polyorganosiloxane compounds comprising quaternary ammonium functions and ester functions may also contain: 1) individual molecules which contain quaternary ammonium functions and no ester functions; 2) molecules which contain quaternary ammonium functions and ester functions; and 3) molecules which contain ester functions and no quaternary ammonium functions. While not limited to structure, the above described polyorganosiloxane compounds comprising quaternary ammonium functions and ester functions are to be understood as mixtures of molecules comprising a certain averaged amount and ratio of both moieties.

Various monofunctional organic acids may be utilized to yield the esters. Exemplary embodiments include C₁-C₃₀ carboxylic acids, for example C₂, C₃, C₈ acids, C₁₀-C₁₈ carboxylic acids, for example C₁₂, C₁₄, C₁₆ acids, saturated, unsaturated and hydroxyl functionalized C₁₈ acids, alkylpolyethercarboxylic acids, alkylsulphonic acids, arylsulphonic acids, alkylarylsulphonic acids, alkylsulphuric acids, alkylpolyethersulphuric acids, phosphoric acid mono alkyl/aryl esters and phosphoric acid dialkyl/aryl esters.

The inventive compositions may in some embodiments be substantially free of or completely free of silicone.

### Rheology Modifier

The composition of the present invention may comprise from 0 wt % to 2 wt %, from 0.05 to 1.5 wt %, from 0.1 to 1.2 wt %, or from 0.1 to 1 wt % of a polymer as a rheology modifier to stabilize the composition.

The polymer may be a gum(s) selected from the group consisting of *Sclerotium* gum, xanthan gum, guar gum, locust bean gum, tragacanth gum, acacia gum, agar gum, algin gum, gellan gum, carob gum, karaya gum biosaccharide gum, calcium carrageenan, potassium carrageenan, sodium carrageenan, potassium alginate, ammonium alginate, calcium alginate and any combination thereof.

The polymer in the composition may be *Sclerotium* gum.

Non-limiting examples of suitable rheology modifiers include water soluble polymers. Examples of water soluble polymers include, but are not limited to (1) vegetable based polymers such as gum Arabic, tragacanth gum, galactan, guar gum, carob gum, karaya gum, carrageenan, pectin, agar, quince seed, algal colloid, starch (rice, corn, potato, or wheat), and glycyrrhizinic acid; (2) microorganism-based polymers such as xanthan gum, dextran, succinoglucan, and pullulan; and (3) animal-based polymers such as collagen, casein, albumin, and gelatin.

Examples of semi-synthetic water-soluble polymers include (1) starch-based polymers such as carboxymethyl starch and methylhydroxypropyl starch; (2) cellulose-based polymers such as methylcellulose, nitrocellulose, ethylcellulose, methylhydroxypropylcellulose, hydroxyethylcellulose, sodium cellulose sulfate, hydroxypropylcellulose, sodium carboxymethylcellulose (CMC), crystalline cellulose, and cellulose powder; and (3) alginate-based polymers such as sodium alginate and propylene glycol alginate. Examples of synthetic water-soluble polymers include (1) vinyl-based polymers such as polyvinyl alcohol, polyvinyl methyl ether-based polymer, polyvinylpyrrolidone, and carboxyvinyl polymer (CARBOPOL 940, CARBOPOL 941; (2) polyoxyethylene-based polymers such as polyethylene glycol 20,000, polyethylene glycol 6,000, and polyethylene glycol 4,000; (3) copolymer-based polymers such as a copolymer of polyoxyethylene and polyoxypropylene, and PEG/PPG methyl ether; (4) acryl-based polymers such as poly(sodium acrylate), poly(ethyl acrylate), polyacrylamide, polyethylene imines, and cationic polymers. The water-swellable clay minerals are nonionic water-soluble polymers and correspond to one type of colloid-containing aluminum silicate having a triple layer structure. More particular, as examples thereof, mention may be made of bentonite, montmorillonite, beidellite, nontronite, saponite, hectorite, aluminum magnesium silicate, and silicic anhydride.

### ADDITIONAL COMPONENTS

The composition of the present invention may include other additional components, which may be selected by the artisan according to the desired characteristics of the final product and which are suitable for rendering the composition more cosmetically or aesthetically acceptable or to provide them with additional usage benefits. Such other additional components generally are used individually at levels of from 0.001% to 10%, preferably up to 5% by weight of the composition.

A wide variety of other additional components can be formulated into the present compositions. These include: other conditioning agents such as aloe vera gel; aloe barbadensis leaf juice; *ecklonia radiata* extract; natural oils and waxes such as shea butter, safflower oil, cocoa butter, orange peel wax, olive oil, macadamia seed oil, *Oenothera biennis* oil, *Crambe abyssinica* see oil, argon oil, camelina oil, sunflower oil, almond oil, *Argania spinosa* kernel oil, grape see oil, jojoba oil, coconut oil, meadowfoam seed oil, neem oil, linseed oil, castor oil, soybean oil, sesame oil, beeswax, sunflower wax, candelilla wax, rice bran wax, carnauba wax, bayberry wax and soy wax; essential oils such as lime peel oil, lavender oil, peppermint oil, cedarwood oil, tea tree oil, ylang-ylang oil and coensage oil which can be used in fragrance; hydrolyzed collagen with tradename Peptein 2000 available from Hormel, vitamin E with tradename Emix-d available from Eisai, panthenol available from Roche, panthenyl ethyl ether available from Roche, hydrolyzed keratin, proteins, plant extracts, and nutrients; pH adjusting agents, such as citric acid, sodium citrate, succinic acid, phosphoric acid, sodium hydroxide, sodium carbonate; salts, in general, such as potassium acetate and sodium chloride; coloring agents, such as any of the FD&C or D&C dyes; perfumes; and sequestering agents, such as disodium ethylenediamine tetra-acetate; and ultraviolet and infrared screening and absorbing agents such as benzophenones, octyl salicylate; antioxidants include: rosemary, tocopherol, vitamin E, vitamin A and tea extracts; amino acids include histidine, 1-arginine and others. preservatives such as sodium benzoate, phenoxyethanol, benzyl alcohol, methyl paraben, propyl paraben, methylchloroisothiazolinone, methylisothiazolinone, disodium ethylenediaminetetraacetic acid (EDTA) and imidazolidinyl urea; and antidandruff agents such as zinc pyrithione, piroctone olamine; non-ionic surfactant such as mono-9-octadecanoate poly(oxy-1,2-ethanediyl) supplied as, for example, Tween 20; and buffer such as aminomethyl propanol, and humectants such as polyhydric alcohols, water soluble alkoxylated nonionic polymers, and mixture thereof.

Some embodiments may be free of any amide polyacrylate.

### PRODUCT FORMS

The compositions of the present invention can be in the form of rinse-off products or leave-on products and can be formulated in a wide variety of product forms, including but not limited to creams, gels, emulsions, mousses, and sprays. The composition of the present invention is especially suitable for hair conditioners, especially leave-on, leave-in, and/or no-rinse hair conditioners. Leave-on and leave-in hair conditioners are generally used on dry, semi-wet, and/or wet hair without rinsing out the conditioner. By no-rinse hair conditioners, what is meant herein is a hair conditioner used on semi-wet to wet hair after shampooing, without rinsing out the conditioner, preferably inside of a bathroom. The conditioning composition of the present invention is especially suitable for rinse-off hair conditioner. Such compositions are preferably used by the following steps: (i) after shampooing hair, applying to the hair an effective amount of the conditioning compositions for conditioning the hair; and (ii) then rinsing the hair.

### EXAMPLES

The following examples further describe and demonstrate embodiments within the scope of the present invention. The examples are given solely for the purpose of illustration and are not to be construed as limitations of the present invention. Where applicable, ingredients are identified by chemical or CTFA name, or otherwise defined below.

### Method of Preparation

The embodiments disclosed and represented by "Ex. 1" through "Ex. 11" are hair conditioning compositions of the present invention which are particularly useful for rinse-off use. Such embodiments have many advantages. For example, they provide improved conditioning on damaged hair without greasy or weighed down hair. Such advantages can be understood by the comparison between the example of the present invention "Ex. 1" through "Ex. 11" and the comparative examples "C1 through C3".

The hair care compositions of "Ex. 1" through "Ex. 11" of the present invention and hair care compositions of "C1" through "C3" as comparative examples can be prepared by any conventional method well known in the art. The inventive compositions have a creamy texture and are not solids or in a solid form.

**Table 1**

| Components | Ex 1 | Ex 2 | Ex 3 | Ex 4 | Ex 5 | Ex 6 |
|---|---|---|---|---|---|---|
| Behenyl trimethylammonium methosulfate | 5.374 | 5.334 | 5.166 | 5.091 | 4.793 | 4.664 |
| Behentrimonium Chloride | | | | | | |
| Cetyl alcohol | 0.073 | 0.086 | 0.140 | 0.164 | 0.259 | 0.297 |
| Stearyl alcohol | 0.196 | 0.231 | 0.376 | 0.441 | 0.698 | 0.805 |
| Citric acid | 0.030 | 0.030 | 0.030 | 0.030 | 0.030 | 0.030 |
| Silicone | 3.500 | 3.500 | 3.500 | 3.500 | 3.500 | 3.500 |
| Preservatives | 0.650 | 0.650 | 0.650 | 0.650 | 0.650 | 0.650 |
| Perfume | 0.700 | 0.700 | 0.700 | 0.700 | 0.700 | 0.700 |
| DI water | QS | QS | QS | QS | QS | QS |
| Wt% ratio of FaOH to CS | 1:20 | 1:16.8 | 1:10 | 1:8 | 1:5 | 1:4.2 |
| Silicone deposition (5 cycles) -Lab treated hair swatches measured by ICP-OES | | | | 99% increase in Si deposition compared to C3 | | 81% increase in Si deposition compared to C3 |
| Silicone deposition (Salon treated harvested hair) - after 3 cycles measured ICP-OES | | | | | | 373% increase in silicone compared to C3 |
| Sebum after 48hrs (GC/FID) - Salon treated harvested hair | | | | | | 44% reduction in sebum compared to C3 |
| Silicone deposition measured from 3-4 cm from tips (salon treated harvested hair) - after 3 cycles measured by FTIR | | | | | | 285% increase in silicone compared to C3 |
| Shear stress at shear rate of 950S⁻¹ | | 110 | | 172 | | 230 |

**Table 1 Cont'd**

| Components | Ex 7 | Ex 8 | Ex 9 | Ex 10 | Ex11 | Ex 12 |
|---|---|---|---|---|---|---|
| Behenyl trimethylammonium methosulfate | 4.382 | 4.200 | 3.953 | 3.734 | | |
| Behentrimonium Chloride | | | | | 4.664 | 4.200 |
| Cetyl alcohol | 0.392 | 0.450 | 0.529 | 0.600 | 0.297 | 0.450 |
| Stearyl alcohol | 1.054 | 1.213 | 1.427 | 1.617 | 0.805 | 1.213 |
| Citric acid | 0.030 | 0.030 | 0.030 | 0.030 | 0.030 | Ex 12 |
| Silicone | 3.500 | 3.500 | 3.500 | 3.500 | 3.500 | 0.030 |
| Preservatives | 0.650 | 0.650 | 0.650 | 0.650 | 0.650 | 3.500 |
| Perfume | 0.700 | 0.700 | 0.700 | 0.700 | 0.700 | 0.650 |
| DI water | QS | QS | QS | QS | QS | 0.700 |
| Wt% ratio of FaOH to CS | 1:3 | 1:2.5 | 1:2 | 1:1.7 | 1:4.2 | 1:2.5 |
| Silicone deposition (5 cycles) -Lab treated hair swatches measured by ICP-OES | | | | | 168% increase in Si deposition compared to C3 | 155% increase in Si deposition compared to C3 |
| Silicone deposition (Salon treated harvested hair) - after 3 cycles measured ICP-OES | | | | | | |
| Sebum after 48hrs (GC/FID) - Salon treated harvested hair | | | | | | |
| Silicone deposition measured from 3-4 cm from tips (salon treated harvested hair) - after 3 cycles measured bv FTIR | | | | | | |
| Shear stress at shear rate of 950S⁻¹ | | 368 | | 300 | 189 | 181 |

**Table 1 Cont'd**

| Components | Cl | C2 | C3 |
|---|---|---|---|
| Behenyl trimethylammonium methosulfate | 2.800 | 0.936 | 2.376 |
| Behentrimonium Chloride | | | |
| Cetyl alcohol | 0.900 | 1.503 | 1.062 |
| Stearyl alcohol | 2.425 | 4.045 | 2.862 |
| Citric acid | 0.030 | 0.030 | 0.030 |
| Silicone | 3.500 | 3.500 | 3.500 |
| Preservatives | 0.650 | 0.650 | 0.650 |
| Perfume | 0.700 | 0.700 | 0.700 |
| DI water | QS | QS | QS |
| Wt% ratio of FaOH to CS | 1:0.8 | 1:0.2 | 1:0.6 |
| Silicone deposition (5 cycles) -Lab treated hair swatches measured by ICP-OES | 18% increase in Si deposition compared to C3 | 46% reduction in Si deposition compared to C3 | |
| Silicone deposition (Salon treated harvested hair) - after 3 cycles measured ICP-OES | | | |
| Sebum after 48hrs (GC/FID) - Salon treated harvested hair | | | |
| Silicone deposition measured from 3-4 cm from tips (salon treated harvested hair) - after 3 cycles measured by FTIR | | | |
| Shear stress at shear rate of 950S⁻¹ | 157 | 183 | 303 |

As can be seen in Table 1, using salon treated harvested hair, the inventive compositions had increased silicone deposition and reduced sebum in relation to the comparative composition. Silicone deposition on hair tips for inventive compositions increased in relation to the comparative composition. Similarly, using lab treated hair swatches, the invention compositions resulted in increased silicone deposition in relation to a comparative composition.

### Test Methods

### Salon treated harvested hair and silicone, sebum measurements

14 panelists with self-perceived oily and damaged hair were recruited for this study. The panelists recruited were split into 2 groups with one group using Comparative Example iii and the other group using Inventive example 6. The panelists were randomized and treated by 2 different stylists throughout the one-week study period. Panelists' hair was treated and harvested by stylists at 4 time point as follows: (a) baseline (after resetting hair with shampoo), (b) after 1^{st} cycle conditioner treatment, (c) 48hrs after treatment and (d) after 3^{rd} cycle conditioner treatment.

### Silicone measurement

Half of the harvested hair was then subjected to extraction using diluted tetrahydrofuran (6% tetrahydrofuran in water). The resulting test solutions were fortified with internal standard and analyzed by inductively coupled plasma - optical emission spectrometry (ICP-OES) against a matrix matched calibration curve made from the silicone of interest. It utilizes microwave digestion to break down hair samples and then determine silicon content using ICP-OES. The result is reported as delta (incremental/reduction) value against the baseline (after shampoo) deposition level in ug/g (n=7 for each measurement).

### Sebum measurement

The other half of the harvested hair was subjected to extraction using 10ml of hexane per 0.1g of hair. The solvent with the hair mixture was vortexed on a pulsed multi-tube vortexer for 5 minutes at 1700 rpm. The above mentioned steps were then repeated with 5ml of hexane per 0.1g of hair. The 2 extracts were dried and combined before reconstituting in 2000 ul hexanes. The resulting test solutions were then subjected to measurements using Gas Chromatography/flame ionization detection (GC/FID). The result is reported as delta (incremental/reduction) value against the baseline (after shampoo) deposition level in ug/g (n=7 for each measurement).

### Silicone deposition at hair tip measured with Fourier Transform Infra-red (FTIR) Spectroscopy

To determine the amount of silicone deposition on the damaged tips, the salon treated harvested hair was measured at 3-4 cm from the tips with mid IR ATR-FTIR (Perkin Elmer Frontier FTIR with Specac Golden Gate single bounce diamond crystal ATR accessory) with 4 replicates each. The measurement range was done from 4000-600cm^{-*1*} in absorption mode, with 8 scans and 4cm⁻*¹* spectral resolution. The ATR clamping pressure was controlled using pressure torque fixed at 70Ncm for all the hair switches measurement. The silicone level can be measured either using chemometric model (Classical Least square and Multi Curve Resolution) which includes the silicones and hair keratin spectrum concentration or with specific unique peak of silicone such as Si-O-Si (975-1150cm^{-*1*}) and Si-CH₃ at (750-900cm^{-*1*}) divided by amide 2 peak area from the hair keratin.

### Lab treated Hair Swatches and silicone measurements.

A swatch of 2 grams of low lift oriental hair at 6 inches length is used for the measurement. Water temperature is set at 37.8°C (100°F) hardness is 7 grain per gallon, and flow rate is 5.68 liter per minute. For shampoo, 0.2g of shampoo is applied onto the center of hair switch and milk for 30 seconds to clean the entire hair length and rinse for 30 seconds. After shampooing, 0.2g of conditioner is applied onto the center of hair switch to cover the entire hair length. The conditioner is spread through the hair switch for 30 seconds followed by 30 seconds of rinsing. The treated hair switch was left to dry in air at 23°C in low humidity (45%) environment overnight. The above-mentioned shampoo and conditioner treatment were repeated for 5 times on the same switch. The treated 2 grams hair swatches are cut into smaller pieces and subjected to extraction for the silicone deposited on the hair surfaces with organic solvent (1:1 hexane/IPA). The extracted silicone solution from the hair is then measured by Inductively Coupled Plasma-Optical Emission Spectroscopy (ICP-OES) (Agilent 5110 synchronous Dual view ICP-OES). The result obtained is reported as Si deposited on hair in ug/g of hair in triplicate (n=3) for each sample.

### Shear Stress

A controlled-stress rotational rheometer (such as Discovery HR-2, TA Instruments, New Castle, Del., USA, or equivalent) capable of sample temperature control (using a Peltier cooler and resistance heater combination) is used for the test. The rheometer is operated in a parallel plate configuration with 40-mm 2° aluminum cone plate tooling. The rheometer is set at 25° C. Approximately 2 ml of sample is gently loaded onto peltier plate using a spatula from the sample jar without any shear to change the product structure, and excess protruding sample is trimmed once the gap reaches 50 µm after sample loading. Sample is then conditioned at 25° C. with 10 s⁻¹ shear rate for 60 seconds and equilibrated at 25° C. for 300 seconds before measurement starts. The test commences with rheometer generating a flow curve by increasing the strain rate from 0.1 s⁻¹ to 1200 s⁻¹ in logarithmic steps. Shear stress at a high shear rate of 950S⁻¹ is measured and extracted using Trios software (provided by TA instrument) and is applicable for other equivalent rheology software.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

The citation of any document is not an admission that it is prior art with respect to any invention disclosed or claimed herein or that it alone, or in any combination with any other reference or references, teaches, suggests or discloses any such invention.

## Claims

1. A hair care composition comprising:
a. a fatty alcohol;
b. a cationic surfactant; and
c. an aqueous base;
wherein the weight ratio of the fatty alcohol to the cationic surfactant is from 1:2 to 1:20; and
wherein the composition is free of any anionic surfactant.

2. The composition of claim 1, wherein the weight ratio of the fatty alcohol to the cationic surfactant is from 1:2 to 1:10.

3. The composition of any preceding claim, wherein the composition comprising at least 50% of the aqueous carrier by weight of the composition.

4. The composition of any preceding claim, wherein the shear stress of the composition is at least 100 Pa, wherein the shear stress is measured using a controlled-stress rotational rheometer like Discovery HR-2, TA Instruments, New Castle, Del., USA, capable of sample temperature control using a Peltier cooler and resistance heater combination, wherein the rheometer is operated in a parallel plate configuration with 40-mm 2° aluminum cone plate tooling, wherein the rheometer is set at 25°C, wherein 2 ml of a sample is gently loaded onto peltier plate using a spatula from the sample jar without any shear to change the product structure, and excess protruding sample is trimmed once the gap reaches 50 µm after sample loading, wherein the sample is then conditioned at 25°C with 10 s⁻¹ shear rate for 60 seconds and equilibrated at 25°C for 300 seconds before measurement starts, wherein a test commences with rheometer generating a flow curve by increasing the strain rate from 0.1 s⁻¹ to 1200 s⁻¹ in logarithmic steps, and wherein the shear stress at a high shear rate of 950S⁻¹ is measured and extracted using Trios software, TA instrument.

5. The composition of any preceding claim, wherein the fatty alcohol is a C12 to C30 saturated fatty alcohol, preferably the fatty alcohol is selected from cetyl alcohol, stearyl alcohol, behenyl alcohol, 2-hexyl-1-decanol, 2-octyl-1-dodecanol and mixtures thereof, more preferably the fatty alcohol is selected from cetyl alcohol, stearyl alcohol, behenyl alcohol, and mixtures thereof.

6. The composition of any preceding claim, wherein the cationic surfactant is present at 0.1% to 25%, by weight of the composition.

7. The composition of any preceding claim, wherein the fatty alcohol is present at 0.05% to 12.5%, by weight of the composition.

8. The composition of any preceding claim, wherein the composition is free of at least one of propellant, silicone and conditioning agents.

9. The composition of any preceding claim, further comprising an acid selected from l-glutamic acid, lactic acid, hydrochloric acid, malic acid, succinic acid, acetic acid, fumaric acid, tartaric acid, citric acid, l-glutamic hydrochloride, maleic acid, salicylic acid, aspartic acid, and mixtures thereof.

10. The composition of any preceding claim, wherein the composition is a leave on hair conditioner.

11. The composition of any preceding claim, wherein the cationic surfactant comprises a C12 to C30 saturated fatty tail, preferably the cationic surfactant is selected from cetyltrimethylammonium chloride, behentrimonium methosulfate, behentrimonium chloride, stearyltrimethylammonium chloride, and mixtures thereof.

12. The composition of any one of claims 1 to 10, wherein the cationic surfactant is an amine, preferably the cationic surfactant is selected from stearamidopropyldimethylamine, behenamidopropyldimethylamine and mixtures thereof.

## Patentansprüche

1. Haarpflegezusammensetzung, umfassend:
a. einen Fettalkohol;
b. ein kationenaktives Tensid; und
c. eine wässrige Base;
wobei das Gewichtsverhältnis des Fettalkohols zu dem kationenaktiven Tensid von 1 : 2 bis 1 : 20 ist; und
wobei die Zusammensetzung frei von beliebigen anionischen Tensiden ist.

2. Zusammensetzung nach Anspruch 1, wobei das Gewichtsverhältnis des Fettalkohols zu dem kationenaktiven Tensid von 1 : 2 bis 1 : 10 ist.

3. Zusammensetzung nach einem der vorstehenden Ansprüche, die Zusammensetzung umfassend mindestens zu 50 Gew.-% der Zusammensetzung den wässrigen Träger.

4. Zusammensetzung nach einem der vorstehenden Ansprüche, wobei die Scherkräfte der Zusammensetzung bei mindestens 100 Pa sind, wobei die Scherkräfte unter Verwendung eines Rotationsrheometer mit kontrollierter Spannung wie Discovery HR-2, TA Instruments, New Castle, Del., USA, gemessen werden, das zu Probentempersteuerung unter Verwendung einer Kombination aus Peltier-Kühlvorrichtung und Widerstandsheizvorrichtung imstande ist, wobei das Rheometer in einer Parallelplattenkonfiguration mit 40-mm 2° Aluminiumkegelplattenwerkzeug betrieben wird, wobei das Rheometer auf 25 °C eingestellt ist, wobei 2 ml einer Probe vorsichtig unter Verwendung eines Spatels aus dem Probengefäß auf die Peltierplatte geladen werden, ohne dass eine beliebige Scherung die Produktstruktur verändert wird, und überschüssige, vorstehende Probe gekürzt wird, sobald der Spalt nach dem Beladen der Probe 50 µm erreicht, wobei die Probe dann bei 25 °C mit 10 s⁻¹ Scherrate für 60 Sekunden und Gleichgewicht bei 25 °C für 300 Sekunden vor Beginn der Messung konditioniert wird, wobei ein Test mit einem Rheometer beginnt, das eine Fließkurve durch ein Erhöhen der Beanspruchungsrate von 0,1 s⁻¹ bis 1200 s⁻¹ in logarithmischen Schritten erzeugt, und wobei die Scherkräfte bei einer hohen Scherrate von 950S⁻¹ unter Verwendung von Trios-Software, TA Instrument, gemessen und extrahiert werden.

5. Zusammensetzung nach einem der vorstehenden Ansprüche, wobei der Fettalkohol ein gesättigter C12 bis C30-Fettalkohol ist, vorzugsweise der Fettalkohol ausgewählt ist aus Cetylalkohol, Stearylalkohol, Behenylalkohol, 2-Hexyl-1-decanol, 2-Octyl-1-dodecanol und Mischungen davon, mehr bevorzugt der Fettalkohol ausgewählt ist aus Cetylalkohol, Stearylalkohol, Behenylalkohol und Mischungen davon.

6. Zusammensetzung nach einem der vorstehenden Ansprüche, wobei das kationenaktive Tensid zu 0,1 Gew.-% bis 25 Gew.-% der Zusammensetzung vorhanden ist.

7. Zusammensetzung nach einem der vorstehenden Ansprüche, wobei der Fettalkohol zu 0,05 Gew.-% bis 12,5 Gew.-% der Zusammensetzung vorhanden ist.

8. Zusammensetzung nach einem der vorstehenden Ansprüche, wobei die Zusammensetzung frei von mindestens einem ist von Treibstoff, Silikon und Konditioniermittel.

9. Zusammensetzung nach einem der vorstehenden Ansprüche, ferner umfassend eine Säure, die aus L-Glutaminsäure, Milchsäure, Salzsäure, Äpfelsäure, Bernsteinsäure, Essigsäure, Fumarsäure, Weinsteinsäure, Zitronensäure, L-Glutaminsäurehydrochlorid, Maleinsäure, Salicylsäure, Asparaginsäure und Mischungen davon ausgewählt ist.

10. Zusammensetzung nach einem der vorstehenden Ansprüche, wobei die Zusammensetzung eine Haarspülung ist, die zum Belassen auf dem Haar bestimmt ist.

11. Zusammensetzung nach einem der vorstehenden Ansprüche, wobei das kationenaktive Tensid einen gesättigten C12 bis C30-Fettschwanz umfasst, vorzugsweise wobei das kationenaktive Tensid aus Cetyltrimethylammoniumchlorid, Behentrimoniummethosulfat, Behentrimoniumchlorid, Stearyltrimethylammoniumchlorid und Mischungen davon ausgewählt ist.

12. Zusammensetzung nach einem der Ansprüche 1 bis 10, wobei das kationenaktive Tensid ein Amin ist, vorzugsweise das kationenaktive Tensid aus Stearamidopropyldimethylamin, Behenamidopropyldimethylamin und Mischungen davon ausgewählt ist.

## Revendications

1. Composition pour soins capillaires comprenant :
a. un alcool gras ;
b. un agent tensioactif cationique ; et
c. une base aqueuse ;
dans laquelle le rapport pondéral de l'alcool gras à l'agent tensioactif cationique est compris entre 1:2 et 1:20 ; et
dans laquelle la composition est exempte de quelconque agent tensioactif anionique.

2. Composition selon la revendication 1, dans laquelle le rapport pondéral de l'alcool gras à l'agent tensioactif cationique va de 1:2 à 1:10.

3. Composition selon l'une quelconque revendication précédente, dans laquelle la composition comprend au moins 50 % du support aqueux, en poids de la composition.

4. Composition selon l'une quelconque revendication précédente, dans laquelle la contrainte de cisaillement de la composition est d'au moins 100 Pa, dans laquelle la contrainte de cisaillement est mesurée à l'aide d'un rhéomètre rotatif à contrainte commandée comme le Discovery HR-2, TA Instruments, New Castle, Del, USA, capable de commander la température d'échantillon à l'aide d'une combinaison de refroidisseur Peltier et d'élément chauffant à résistance, dans laquelle le rhéomètre est utilisé dans une configuration de plaque parallèle avec un outillage de plaque conique en aluminium de 40 mm à 2 °, dans laquelle le rhéomètre est réglé à 25 °C, dans laquelle 2 ml d'un échantillon sont délicatement chargés sur la plaque Peltier à l'aide d'une spatule à partir du pot d'échantillon sans aucun cisaillement pour changer la structure du produit, et l'échantillon en saillie excédentaire est taillé une fois que l'écart atteint 50 µm après le chargement d'échantillon, dans laquelle l'échantillon est ensuite conditionné à 25 °C avec un taux de cisaillement de 10 s⁻¹ pendant 60 secondes et équilibré à 25 °C pendant 300 secondes avant le début de la mesure, dans laquelle un test commence avec le rhéomètre générant une courbe d'écoulement en augmentant le taux de déformation de 0,1 s⁻¹ à 1 200 s⁻¹ par étapes logarithmiques, et dans laquelle la contrainte de cisaillement à un taux de cisaillement élevé de 950 s⁻¹ est mesurée et extraite à l'aide du logiciel Trios, de l'instrument TA.

5. Composition selon l'une quelconque revendication précédente, dans laquelle l'alcool gras est un alcool gras saturé en C12 à C30, de préférence, l'alcool gras est choisi parmi l'alcool cétylique, l'alcool stéarylique, l'alcool béhénylique, le 2-hexyl-1-décanol, le 2-octyl-1-dodécanol et des mélanges de ceux-ci, plus préférablement, l'alcool gras est choisi parmi l'alcool cétylique, l'alcool stéarylique, l'alcool béhénylique, et des mélanges de ceux-ci.

6. Composition selon l'une quelconque revendication précédente, dans laquelle ledit agent tensioactif cationique est présent en une quantité de 0,1 % à 25 % en poids de la composition.

7. Composition selon l'une quelconque revendication précédente, dans laquelle l'alcool gras est présent à hauteur de 0,05 % à 12,5 %, en poids de la composition.

8. Composition selon l'une quelconque revendication précédente, dans laquelle la composition est exempte d'au moins un des agents propulseurs, de silicone et d'après-shampooing.

9. Composition selon l'une quelconque revendication précédente, comprenant en outre un acide choisi parmi l'acide l-glutamique, l'acide lactique, l'acide chlorhydrique, l'acide malique, l'acide succinique, l'acide acétique, l'acide fumarique, l'acide tartrique, l'acide citrique, le chlorhydrate de l-glutamique, l'acide maléique, l'acide salicylique, l'acide aspartique, et des mélanges de ceux-ci.

10. Composition selon l'une quelconque revendication précédente, dans laquelle la composition est un après-shampooing pour cheveux.

11. Composition selon l'une quelconque revendication précédente, dans laquelle le tensioactif cationique comprend une queue grasse saturée en C12 à C30, de préférence, le tensioactif cationique est choisi parmi le chlorure de cétyltriméthylammonium, le méthosulfate de béhentrimonium, le chlorure de béhentrimonium, le chlorure de stéaryltriméthylammonium, et leurs mélanges.

12. Composition selon l'une quelconque des revendications 1 à 10, dans laquelle l'agent tensioactif cationique est une amine, de préférence, l'agent tensioactif cationique est choisi parmi la stéaramidopropyldiméthylamine, la béhénamidopropyldiméthylamine et des mélanges de ceux-ci.
